(19)

Europäisches
Patentamt
European
Patent Office
Office européen
des brevets

(11) **EP 2 636 428 A1**

(12) **EUROPEAN PATENT APPLICATION**

(43) Date of publication:
**11.09.2013 Bulletin 2013/37**

(51) Int Cl.:
*A61N 7/02* *(2006.01)*

(21) Application number: **12305278.9**

(22) Date of filing: **08.03.2012**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**

(72) Inventors:
• **Chapelon, Jean-Yves**
  **69100 Villeurbanne (FR)**
• **Lafon, Cyril**
  **69780 Toussieu (FR)**
• **Mestas, Jean-Louis**
  **38630 Veyrins-Thuellin (FR)**

(71) Applicant: **INSERM (Institut National de la Santé et de la Recherche Médicale)**
**75013 Paris (FR)**

(74) Representative: **Chevalier, Renaud Philippe**
**Cabinet GERMAIN & MAUREAU**
**12, rue Boileau**
**BP 6153**
**69466 Lyon Cedex 06 (FR)**

(54) **Method for determining parameters to generate ultrasound intensity and device for the same**

(57)    The method determines parameters to generate confocal ultrasound beams inside a medium, and uses a device comprising first and second ultrasound transducers (11, 12) and first and second displacement members (13, 14) for moving the transducers. The parameters include signals to the transducers, and the positions of the transducers. The parameters are optimised for having a minimum amplitude of the signal and having an acoustic effect inside the medium.

FIG. 1

EP 2 636 428 A1

**Description**

**FIELD OF THE INVENTION**

The present invention concerns a method and device for determining parameters to generate ultrasound intensity localised inside a medium.

**BACKGROUND OF THE INVENTION**

The invention concerns more precisely a method for determining parameters to generate ultrasound intensity localised inside a region of a medium.
It is known to generate and to focus a single ultrasound beam to a point inside a medium, so that to produce cavitation inside a region around said point.
This method is satisfactory, but still need to be improved.
In the document WO2008/018019 two ultrasound beams are focused.
However, the position of the transducers and the signals sent to the transducers are not optimised. The beams are not accurately superposed at the acoustic transducers focal points.

**OBJECTS AND SUMMARY OF THE INVENTION**

One object of the present invention is to provide a method that accurately tunes the parameters of an ultrasound device.
To this effect, the method uses:

- a support member,
- first and second transducers linked to said support member and adapted to generate first and second focused beams of ultrasound waves inside said medium in response to respective first and second signals, said beams being directed towards respective beams directions that are substantially coplanar and inclined relative to each other of an angle comprised between 60° and 120°, and that intersects each other at an intersection point inside the medium, said first and second transducers being different transducers, separated from each other, and
- first and second displacement members adapted to move at least one of the first and second transducers relative to the other transducer to a position determined by the first and second displacements of the respective first and second displacement members, said displacements being along different displacement directions,

**wherein** the parameters comprises the first and second signals and the first and second displacements, and
**wherein** said parameters are optimised so that the first and second signals have an amplitude that is minimum to generate an acoustic effect, said acoustic effect being localised only inside the region of the medium around the intersection point of the beams directions and not localised outside of the region.
Each transducer has an acoustic focal distance that is a distance between the transducer surface and an acoustic focal point where the ultrasound pressure in the medium has the greater amplitude. However, the transducers have a nonlinear behaviour. The acoustic focal distance depends on the signal amplitude provided to the transducer, and, for example, the acoustic focal distance decrease for high amplitude signals.
It is then difficult to superpose the two acoustic focal points of two separate transducers. The transducers must be moved accordingly.
Thanks to the method, the parameters (signals provided to the transducers and positions of the transducers) are accurately tuned to superpose the acoustic focal points of the transducers so as to have an acoustic effect (e.g. a cavitation effect) inside a region around the intersection point and with minimum amplitude of the signals.
Thanks to these features, the region has a reduced volume and size. The acoustic effect (e.g. cavitation) is localised only inside the region and not outside of this small region. Moreover, the cavitation inside the region is more stable.
In various embodiments of the method, one and/or other of the following features may optionally be incorporated:

- the method further comprises the following successive optimisation steps:

    **a)** providing initial parameters to move the first and second transducers by means of the first and second displacement members to a position and to generate first and second beams by means of said first and second transducers so that an acoustic effect is detected inside the medium,
    **b)** reducing an amplitude of the signals down to an amplitude level where the acoustic effect is not detected,

**c)** moving the first and second transducers by means of the first and second displacement members to a plurality of test positions, each of said test positions corresponding to said position of the transducers wherein at least one of the first and second displacements is modified by a displacement increment,

**d)** if the acoustic effect is detected at one of the test positions at step c), selecting said test position to be the position of the transducers, and repeating steps b) and c),

**e)** the determined parameters are the signals and displacements tuned before the last reduction at step b);

- the method comprises between step d) and e) a step wherein if the displacement increment is not lower or equal than a minimum displacement increment, the displacement increment is reduced and the method is repeated steps b) and c);
- the minimum displacement increment is equal to 0.1 mm, and preferably equal to 0.05 mm;
- the displacement increment is equal to 0.5 mm, and preferably equal to 0.1 mm;
- the first and second signals have equal amplitudes;
- the step b) is implemented by an iterative process wherein the amplitude of the signals is reduced at each loop by a division of the previous amplitude with a reduction factor having a value comprised between one and two, said loop being repeated until the acoustic effect is not detected;
- the reduction factor is lower than 1.5, and preferably lower than 1.1;
- the acoustic effect is an effect chosen in a list comprising a cavitation effect localised inside the region of the medium, and a single acoustic fountain effect for at least two beams and localised at an interface between a first and a second fluid of the medium, and a thermal increase effect.

Another object of the invention is to provide a device for generating ultrasound intensity localised inside a region of a medium, wherein the device comprises:

- a support member,
- first and second transducers linked to said support member and adapted to generate first and second focused beams of ultrasound waves inside said medium in response to respective first and second signals, said beams being directed towards respective beams directions that are substantially coplanar and inclined relative to each other of an angle comprised between 60° and 120°, and that intersects each other at an intersection point inside the medium, said first and second transducers being different transducers, separated from each other, and
- first and second displacement members adapted to move at least one of the first and second transducers relative to the other transducer to a position determined by the first and second displacements of the respective first and second displacement members, said displacements being along different displacement directions,

**wherein** the parameters comprises the first and second signals and the first and second displacements, and
**wherein** the device further comprises a control unit adapted to provide the parameters to move the first and second transducers by means of the first and second displacement members to a position and to generate first and second beams by means of said first and second transducers, and
**wherein** the parameters are optimised so that the first and second signals have an amplitude that is minimum to generate an acoustic effect, said acoustic effect being localised only inside the region of the medium around the intersection point of the beams directions and not localised outside of the region.

In various embodiments of the device, one and/or other of the following features may optionally be incorporated:

- the control unit further comprises a memory and determines the parameters from at least a predetermined focal distance of a transducer stored into the memory for a plurality of signal amplitude;
- the control unit determines the parameters from a predetermined mathematical model of a focal distance of a transducer versus amplitude of a signal provided to the transducer;
- the first and second transducers are high intensity focused ultrasound transducers;
- the device further comprises a sensor adapted to detect the acoustic effect inside the medium;
- the sensor is chosen in a list comprising an hydrophone sensor, a piezoelectric sensor, a fibre optic hydrophone, a camera sensor, a thermal sensor, and a camera sensor;
- the angle is comprised between 70° and 110°;
- each of the displacement directions are perpendicular to one of the beam directions;
- the region has a size determined by the parameters, said size being smaller than 5 mm in all directions, and preferably smaller than 2 mm in all directions;
- the control unit determines the parameters to generate an ultrasound pressure inside the region of the medium executing the following optimisation steps:

**a)** providing initial parameters to move the first and second transducers by means of the first and second displacement members to a position and to generate first and second beams by means of said first and second transducers so that an acoustic effect is detected inside the medium,

**b)** reducing an amplitude of the signals down to an amplitude level where the acoustic effect is not detected,

**c)** moving the first and second transducers by means of the first and second displacement members to a plurality of test positions, each of said test positions corresponding to said position of the transducers wherein at least one of the first and second displacements is modified by a displacement increment,

**d)** if the acoustic effect is detected at one of the test positions at step c), selecting said test position to be the position of the transducers, and repeating steps b) and c),

**e)** the determined parameters are the signals and displacements tuned before the last reduction at step b);

- the acoustic effect is an effect chosen in the list comprising a cavitation effect localised inside the region of the medium, and a single acoustic fountain effect for at least two beams and localised at an interface between a first and a second fluid of the medium, and a thermal increase effect.

According to various aspects, the device is adapted for delivering a substance inside a medium.

It is known to generate and to focus a single ultrasound beam to a point inside a medium, so that to produce cavitation inside a region around said point and to make a delivery compound to switch from a holding state in which the substance is held by the delivery compound, to a releasing state in which the substance is released by the delivery compound.

The device is a more efficient device for delivering a substance inside a medium.

To this effect, the substance is held by a delivery compound in a holding state, and the device comprises:

- first ultrasound means adapted to generate a first beam of a first ultrasound wave inside said medium towards a first direction, said first beam being directed to a target point inside said medium,
- second ultrasound means adapted to generate a second beam of a second ultrasound wave inside said medium towards a second direction, said second beam being substantially directed to said target point, and

wherein the second direction is inclined relative to the first direction for inducing the cavitation inside a region around said target point and for switching the delivery compound from the holding state to a releasing state inside said region.

Thanks to these features, the region wherein the substance is released and delivered has a reduced volume and size. Moreover, the cavitation inside said region is increased compared to the cavitation outside said region. Moreover, the inventors have observed that the cavitation inside the region is more stable.

In various embodiments of the device, one and/or other of the following features may optionally be incorporated:

- the region has a size in all directions lower than 5 mm, and preferably lower than 2 mm;
- the device further comprises third ultrasound means adapted to produce acoustic images of the medium;
- the third ultrasound means produce an image of the medium to define a region of interest inside said medium, and the device comprises control means to move sequentially the target point to a plurality of determined points inside said region of interest for controlling the cavitation inside said determined region of interest;
- the first beam is focused to the target point, the second beam is substantially focused to the target point, and the target point is a focal point;
- the second direction is inclined relative to said first direction of an angle, said angle being comprised between 30° and 150°, and preferably between 60° and 120°;
- the first and second ultrasound waves have different frequencies;
- the first and second ultrasound waves have frequencies comprised between 100 kHz and 10 MHz, and preferably between 0.2 MHz and 1.5 MHz;
- the delivery compound is chosen in the list of a nanoparticle and a microparticule;
- the delivery compound is chosen in the list of a liposome and a micelle.

Another object is to provide a method for delivering a substance inside a medium, wherein the substance is held by a delivery compound in a holding state, and the method comprises the steps of:

- generating by first ultrasound means, a first beam of a first ultrasound wave inside said medium towards a first direction, said first beam being directed to a target point inside said medium,
- generating by second ultrasound means, a second beam of a second ultrasound wave inside said medium towards a second direction, said second beam being substantially directed to said target point, and

wherein the second direction is inclined relative to the first direction for inducing the cavitation inside a region of the medium around said target point and for switching the delivery compound from the holding state to a releasing state inside said region.

In preferred embodiments of the method, one and/or the other of the following features may optionally be incorporated:

- the method further comprises the steps of:

    - imaging the medium to determine a region of interest inside the medium,
    - moving sequentially the target point to a plurality of determined points inside the region of interest;

- each determined point inside the region of interest is controlled with a predetermined elapsed time or predetermined intensity of said first and second beams;
- the medium comprises cells, the substance comprises at least a plasmid, the target point is positioned near at least one cell, so that the cavitation releases the substance and open the one cell to transfer the plasmid inside said cell;
- the substance is a drug intended to be delivered to a tumor inside said medium, and the target point is positioned inside said tumor;
- the medium comprises fat cells, said fat cells being substantially destroyed by positioning the target point on these fat cells;
- the first beam is focused to the target point, the second beam is substantially focused to the target point, and the target point is a focal point.

## BRIEF DESCRIPTION OF THE DRAWINGS

Other features and advantages of the invention will be apparent from the following detailed description of embodiments given by way of non-limiting examples, with reference to the accompanying drawings. In the drawings:

- Figure 1 and 2 are schematic views of a device for generating a localised ultrasound pressure according to the invention,
- Figure 3 is a perspective view of an embodiment of the device of figure 2,
- Figures 4a and 4b are flowcharts of an embodiment of an optimisation method to determine the parameters.

## MORE DETAILED DESCRIPTION

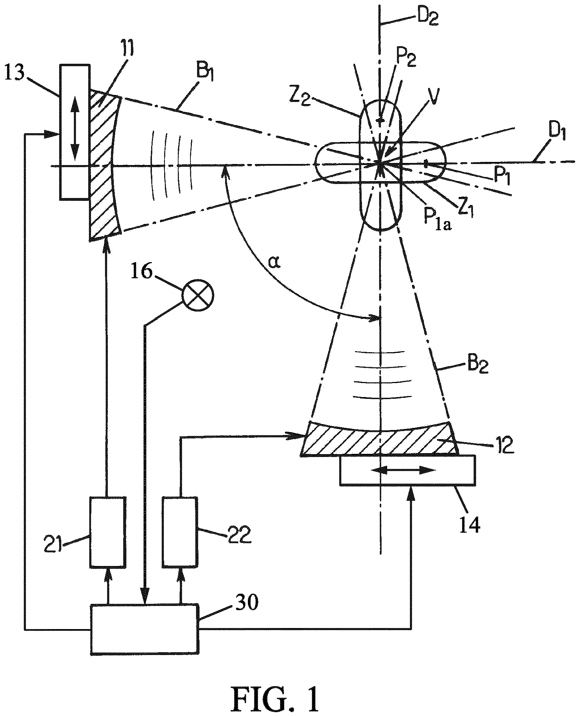

**Figure 1** represents a first embodiment of a **device** 1 generating a localised ultrasound pressure that can be used also for delivering a substance inside a medium.

The medium may be any medium that can propagate ultrasound waves and have cavitation phenomenon. For example, the medium may be an aqueous medium. The medium may be a human or animal body. The medium may be also a aqueous medium enclosed inside a test tube or a test capsule or any test device. Therefore, the medium may be an in-vitro or an in-vivo medium.

The medium is susceptible to the cavitation phenomenon. The cavitation is the creation or formation of vapour bubbles in a medium in a region where the pressure falls bellow a pressure threshold, said threshold corresponding to the called cavitation level. Additionally, said pressure threshold may be the vapour pressure of said medium or higher to this vapour pressure. During cavitation the cavitation bubbles may dynamically oscillates. Then, the cavitation bubbles may rapidly collapse, producing a localised shock wave in the medium, an increase of local temperature, some mechanical stresses and/or chemical effects. The cavitation may be produced when the medium comprises some impurities. The cavitation may be also produced by adding microbubbles or bubbles inside the medium, or by adding ultrasound contrast agents.

The substance may be a chemical substance, a radioactive substance, a colour substance, a gene, a plasmide or a drug.

The substance is held by a delivery compound in a holding state when there is no cavitation inside the medium in near proximity of the delivery compound. In that state, the substance is not free to move into the medium independently of the delivery compound, and is not able to be active and to react with another substance inside the medium.

The substance is released by the delivery compound in a releasing state when there is cavitation inside the medium in near proximity of the delivery compound. In that state, the substance is free to move into the medium independently of the delivery compound, and is able to be active and to combine with other substance.

The delivery compound is therefore sensible to the cavitation, and releases the substance if it is in near proximity of cavitation bubbles. Near proximity means a distance between the delivery compound and a cavitation bubble smaller than 1 mm.

If cavitation bubbles are localized inside a region V of the medium, i.e. if the cavitation occurs inside said region V, the delivery compound releases the substance substantially inside said region V. The substance is transported through the medium to the localized region V inside said medium and released inside said localized and reduced region V.

The delivery compound may be considered as a means of transportation and delivering for the substance inside the medium from any origin to the localized region V.

Many delivery compounds are well-known. It may be a microparticules or nanoparticle, and for example a liposome, a micelle, or the like.

As used herein, the term "particles" refers to an aggregated physical unit of solid material. The particles according to the invention may be micro- or nanoparticles.

Microparticles are understood as particles having a median diameter $d_{50}$ ranging from 500 $\mu$m to 1 $\mu$m and more preferably from 100 $\mu$m to 1 $\mu$m, and most preferably from 10 $\mu$m to 1 $\mu$m.

Nanoparticles are understood as particles having a median diameter $d_{50}$ inferior to 1 $\mu$m and notably ranging from 0.1 $\mu$m and 0.01 $\mu$m.

As used herein, the term "median diameter $d_{50}$" refers to the particle diameter so that 50 % of the volume or of the number of the particles population have a smaller diameter.

More specifically, the microparticles or nanoparticles may be microspheres or microcapsules, nanospheres or nanocapsules respectively, containing an active substance.

The device 1 comprises:

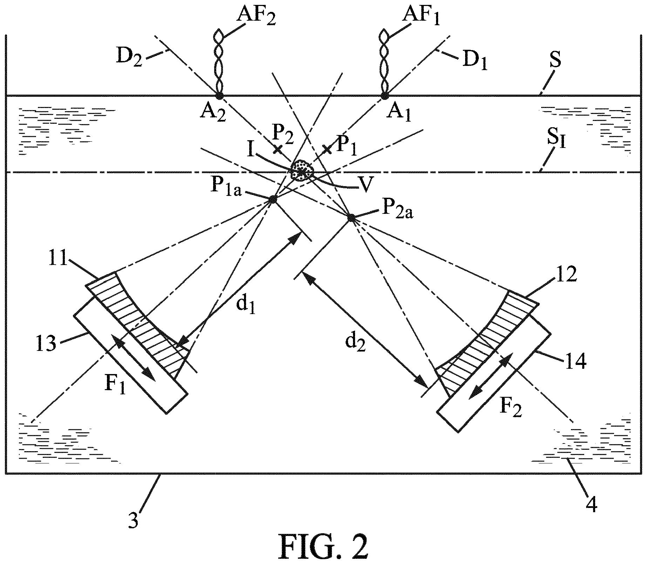

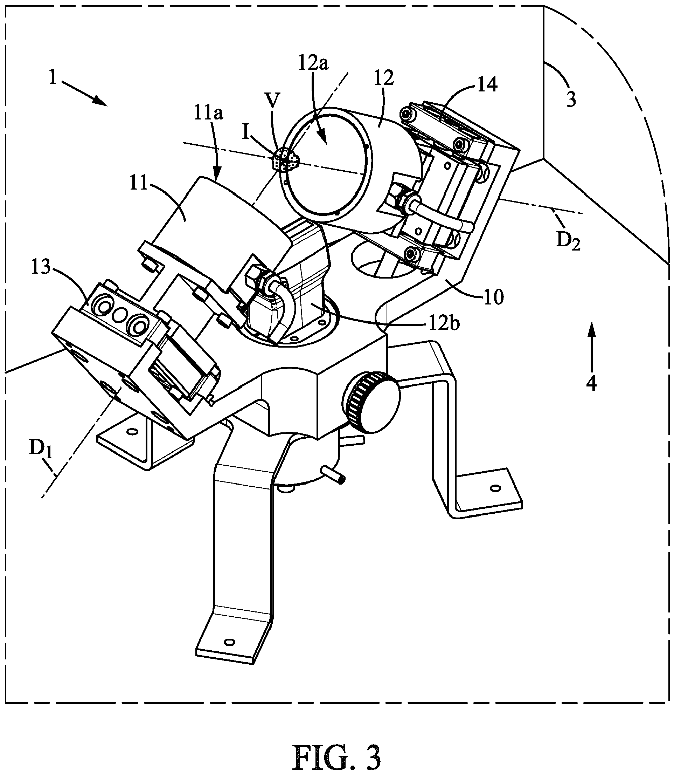

- a support member 10 (see **figure 3),**
- first ultrasound means 11 (first transducer) linked to said support member 10, and adapted to generate a first beam $B_1$ of a first ultrasound wave inside the medium towards a first beam direction $D_1$, said first beam $B_1$ being focused to a first acoustic focal point $P_{1a}$ inside said medium, and
- second ultrasound means 12 (second transducer) linked to said support member 10, and adapted to generate a second beam $B_2$ of a second ultrasound wave inside the medium towards a second beam direction $D_2$, said second beam $B_2$ being substantially focused to a second acoustic focal point $P_{2a}$ (not shown) at a distance from said first acoustic focal point $P_{1a}$ lower than 5 mm, and preferably lower than 1 mm. For simplicity, the first and second acoustic focal points $P_{1a}$ and $P_{2a}$ are supposed superposed on the figure 1, but are visible on figure 2.

The first and second beams directions $D_1$, $D_2$ are substantially coplanar and inclined relative to each other of an angle comprised between 60° and 120°. These beams directions $D_1$, $D_2$ intersects each other at an intersection point I inside the medium (see **figure 2).**

The angle may be comprised between 70° and 110°. According to the performed experiments, this range of angle gives the most reduced size of region V.

For a range of angle between 90° and 110° the inventors discovered that the crossing of first and second beams $B_1$, $B_2$ additionally generates an interference phenomenon that seems to stabilise the position of the cavitation bubbles inside the region V. Such range is preferred.

The first and second transducers 11, 12 are different transducers: They are not transducers elements integrated inside a single transducer. They are each driven by an independent signal to generate an independent ultrasound beam.

The first and second transducers 11, 12 are separated from each other. This is necessary to have a volume between them that is enough to place a test sample. For example, the transducers are separated of a distance greater than 50 mm. This distance may be greater than 100 mm. However, as the transducers are inclined, the said distance can be very small, and null.

The acoustic focal point $P_{1a}$ (or $P_{2a}$) of focused beams is the effective acoustic focal point, that is to say the point into the medium where a pressure i reaches a maximum value, i.e. where the acoustical power or intensity inside the medium has a maximum value.

The first and second transducers 11, 12 are high intensity focused ultrasound (HIFU) transducers. Such transducers are able to generate high level of acoustical power, and are able to generate very small pressure and cavitation in the medium.

The first ultrasound means 11 comprises at least a first transducer receiving a first signal $S_1$ from a first signal generator 21 and generating the first beam $B_1$.

The second ultrasound means 12 comprises at least a second transducer receiving a second signal $S_2$ from a second signal generator 22 and generating the second beam $B_2$.

The device 1 also comprises control means or a control unit 30 adapted to control, synchronise and tune the characteristics of the first and second signal generators 21, 22. The control unit 30 may be a computer with a keyboard, and a display for interfacing with a user.

The first signal $S_1$ and the second signal $S_2$ may be time continuous signals, or burst signals or a combination of continuous and burst sine signals. These signals comprise for example a sine wave or a plurality of sine waves or any other wave.

In case of first and second burst signals, the control unit 30 synchronises these first and second signals so that the first and second waves arrive near the focal point P at the same time. The waves add their amplitudes. The local pressure amplitude (acoustic power or intensity) may be increased.

The first and second transducers also have a geometric focal point, respectively marked with the references $P_1$ and $P_2$ on figure 1. These geometric focal points are localized at a predetermined geometric focal distance from a surface of each transducer, said surface producing the ultrasound wave into the medium.

The geometric focal point $P_1$ (or $P_2$) is defined by the geometrical properties of the transducer. For example, for a transducer having a semi-spherical surface, the geometric focal distance is substantially equal to the radius of curvature of said transducer's surface.

The acoustic focal point $P_{1a}$ ($P_{2a}$ respectively) is located near the geometric focal point $P_1$ ($P_2$ respectively) for a small amplitude signal provided to the transducer. Due to the nonlinear behaviour of the transducer, this acoustical focal point is moving towards the transducer's surface with respect to increase of the signal amplitude.

As shown on figure 1, the geometric focal points $P_1$, $P_2$ are usually situated on the respective direction, first or second direction $D_1$, $D_2$, and they are situated at a distance from the transducer surface preferably higher than the acoustic focal point $P_{1a}$, $P_{2a}$ of the ultrasound beams $B_1$, $B_2$.

For a high amplitude signal, the acoustical focal point of a transducer is away from or distant from the geometrical focal point: A distance that can be higher than 5 mm. It is therefore difficult to superpose the acoustic focal points of a plurality of transducers, and even for only two transducers.

The first beam $B_1$ produces a first zone $Z_1$ of low pressure inside the medium, i.e. a first zone of maximum acoustic power or intensity. The first zone $Z_1$ is typically centred on the acoustic focal point $P_{1a}$ and has an elongated shape along the first direction $D_1$. The first zone $Z_1$ is for example a region of the medium having a length of 13 mm in the first direction $D_1$ and a width of 2 mm in orthogonal directions perpendicular to the first direction $D_1$.

The second beam $B_2$ produces a second zone $Z_2$ of low pressure inside the medium, i.e. a second zone of maximum acoustic power or intensity. The second zone $Z_2$ is substantially centred on the acoustic focal point $P_{2a}$ and has an elongated shape along the second direction $D_2$. The second zone $Z_2$ is similar to the first zone $Z_1$, and it has for example the same size as the first zone $Z_1$, but it is elongated in the second direction $D_2$.

In case of a single beam focused to the acoustic focal point $P_{1a}$, the first zone $Z_1$ corresponds to a region of the medium wherein the cavitation occurs if the first signal $s_1$ has an amplitude greater than a predetermined amplitude. This first zone is quite large and elongated. Moreover, the cavitation and cavitation thresholds inside this region are not stable: Cavitation bubbles appear and collapse at various positions inside the volume. These positions of the cavitation bubbles seem to move inside the region, and not to be equally spatially spread inside said region.

In case of non coaxial and confocal dual beams, the first and second zones $Z_1$, $Z_2$ intersect in a region V around the focal point P, said region V having a reduced size compared to the size of said first zone $Z_1$ or said second zone $Z_2$.

Thanks to these features, the region V has a reduced size and the cavitation inside this region V is increased compared to the cavitation outside said region.

For example, the region V may have a size in all directions lower than 5 mm, and preferably lower than 2 mm.

The second direction $D_2$ is inclined relative to the first direction $D_1$ of an angle α. The second direction D2 is therefore non coaxial to the first direction D1. The angle can be for example of 90°. The region V has therefore the smallest size.

The angle α may be comprised between 30° and 150°, and preferably between 60° and 120°. A reduced angle α may be useful to have more than two ultrasound means in the device, to define a more precise shape of the region V, to reduce the size of the region V and to better stabilize the bubbles inside said region V.

If the first signal $s_1$ has an amplitude greater than a first predetermined amplitude and if the second signal $s_2$ has an amplitude greater than a second predetermined amplitude, the cavitation occurs inside the region V that is substantially the intersection of the first and second zones $Z_1$, $Z_2$, and there are no cavitation outside of said region V.

The first signal $s_1$ has an amplitude tuned so that the first beam does not induce the cavitation near the acoustic focal point $P_{1a}$ in the absence of the second beam. Reciprocally, the second signal $s_2$ has an amplitude tuned so that the second beam does not induce the cavitation near the acoustic focal point $P_{2a}$ in the absence of the first beam. But, the amplitude of the first and second signals are tuned so that to induce the cavitation inside the region V around the superposed acoustic focal points $P_{1a}$, $P_{2a}$, when both beams are present or at least when both of the first and second waves arrive inside said region V of the medium.

The first and second signals may comprise same or different frequencies, to respectively produce first and second ultrasound waves.

Usually, the signals and waves have main frequencies comprised between 100 kHz and 10 MHz. Preferably, these frequencies are comprised between 0.2 MHz and 1.5 MHz.

Inside the region V, the delivery compound switches from the holding state to the releasing state. The substance is released and delivered inside said region V.

**Figure 3** represents a more detailed view of an embodiment of the device 1 comprising a water tank 3 filled with a medium 4 of degassed water. The degassed water is a medium having cavitation pressure levels equivalent to in vivo medium. It is therefore representative for tuning the parameters of the device. A non-degassed water would lead to lots of cavitation bubbles inside the medium, and inaccurate tuning of the device.

In this device 1, the first ultrasound means comprises a spherical transducer 11 having an emitting surface 11a for emitting the first ultrasound wave in the first direction $D_1$ and for focusing the first ultrasound wave at a first distance from the emitting surface 11a.

The first transducer 11 is a piezoelectric ceramic transducer having a nominal frequency of 1 MHz, a first distance of 50 mm and an emitting surface 11a of 50 mm diameter.

The second ultrasound means comprises a second transducer 12 having an emitting surface 12a for emitting the second ultrasound wave in the second direction D2 and for focusing the second ultrasound wave at a second distance from the emitting surface 12a.

The device 1 optionally comprises an imaging transducer 12b in order to produce images of the medium 4.

The images produced with the imaging transducer 12b are used to visualize the medium 4 and to localize the cavitation bubbles inside the medium 4. These images may be used to determine the first and second signal amplitudes to control the cavitation level, in such a way as a closed controlled loop.

The second transducer 12 is for example identical to the first one. But, it can be different.

The imaging transducer 12b is for example a transducer, having a nominal frequency of 5 MHz and a comprising multi-element array of piezoelectric elements.

The first signal $s_1$ is a pulsed burst sine wave of 1 MHz, with repetition frequency of 200 Hz, and a duty cycle of 5 %. The first predetermined amplitude corresponds to a power of generated first ultrasound wave of 5 Watts.

In this embodiment, designed for testing, the angle $\alpha$ between the first direction $D_1$ and the second direction $D_2$ is substantially of 110°.

The tests performed with this device 1 confirmed that the region V where the cavitation is produced is a small region having a size approximately of 2 mm x 2 mm x 2 mm in three orthogonal directions (X, Y, Z).

Such region V having cavitation bubbles is observed with the imaging transducer 12b at a position relative to the device 1 that is stable, not moving in time during.

Inside said region V, the cavitation bubbles have in time during a constant density and are uniformly spread inside the region V.

Outside said region V, no cavitation is observed.

The device 1 further comprises first and second displacement members 13, 14 adapted to move the first and second transducers 11, 12 to a position determined by a first and second displacements $x_1$, $x_2$ of said respective first and second displacement members.

The first displacement member 13 may support and be connected to the first transducer 11 for moving it according to a first displacement direction. Reciprocally, the second displacement member 14 may support and be connected to the second transducer 12 for moving it according to a second displacement direction. The first and second displacement members can move each transducer independently to the other.

According to a variant, the first and second displacement members may support only one of the transducers 11, 12. For example, the first transducer 11 is fixed to the support member 10, and the other of the transducers can be moved according to the two displacement directions (represented by arrows $F_1$ and $F_2$ on the figures).

The displacement directions are different from each other. The transducers can be moved in the plane of the first and second beams. The displacement directions can be perpendicular to the beam directions $D_1$, $D_2$. For example, displacement direction of the first displacement member 13 may be perpendicular to the first beam direction $D_1$, and the displacement direction of the second displacement member 14 may be perpendicular to the second beam direction $D_2$.

In case, of two transducers (11, 12), the device 1 only needs two displacements members (13, 14) to superpose the first and second acoustic focal points $P_{1a}$, $P_{2a}$. Generally, if the beams directions are coplanar, to superpose all the acoustic focal points, the device 1 needs to comprise a number of displacement members that is equal to 2.(N-1), where N is the number of transducers.

The control unit 30 provides the first and second signals $s_1$, $s_2$ to the first and second transducers 11, 12 respectively to generate the beams. It also provides the first and second displacements $x_1$, $x_2$ to the first and second displacement members respectively to move the transducers.

These first and second signals $s_1$, $s_2$ (mainly their respective amplitudes) and the first and second displacements $x_1$, $x_2$ are therefore parameters for tuning the device 1.

**Figure 2** shows a configuration of the device 1 that is not correctly tuned. The first and second acoustic focal points $P_{1a}$, $P_{2a}$ are not superposed to each other and are not superposed on the intersection point I. But this figure helps to understand how the device 1 is operated to superpose said acoustic focal points.

On this figure, a first focal distance $d_1$ of the first transducer 11 is presented. This distance is the distance from the transducer surface to the first acoustic focal point $P_{1a}$. A second focal distance $d_2$ of the second transducer is also presented.

The first and second beams directions $D_1$, $D_2$ intersect at an intersection point I, inside the medium.

Around the intersection point I, a region V is virtually delimited on the figure 2. Inside the region V, the first and second beams $B_1$, $B_2$ may generate an acoustic effect inside said region V; said acoustic effect is a cavitation effect.

On the figure, the medium comprises a liquid having an upper surface S between said liquid and air. The liquid is contained inside a tank 3. The level of the liquid surface can be changed by liquid control means. For example, the level of the liquid can be lowered to a lower level. For example, the level of the liquid can be tuned to a specific level $S_l$ wherein the intersection point I is near or above said specific level $S_l$ of the liquid.

The first beam direction $D_1$ intersects the surface S at a first interface point $A_1$. Respectively, the second beam direction $D_2$ intersects the surface S at a second interface point $A_2$.

Each of the first and second beams $B_1$, $B_2$, generates acoustic radiation pressure inside the medium. Said acoustic radiation pressure may generate an acoustic effect at the liquid surface, called the acoustic fountain: Some droplets of liquid can be projected into air above the fluid surface S from the interface point. The first acoustic fountain $AF_1$ and second acoustic fountain $AF_2$ are represented on the figure 2. Such acoustic fountains can easily be detected by an optical sensor, e.g. a camera sensor.

This acoustic fountain effect can be used to determine the exact position of the first and second interface points $A_1$, $A_2$, that is to say the positions of the first and second beams $B_1$, $B_2$. The displacement members 13, 14 can therefore be actuated according to lateral directions so as the first and second beam directions are correctly coplanar.

In case, the liquid level is the specific level $S_l$, both acoustic fountains are superposed: only one acoustic fountain is visible.

The acoustic fountain effect can then be used to determine experimentally the exact position of the intersection point I, and acoustic focal points.

The first and second focused beams $B_1$, $B_2$, also generate an increase of temperature at the acoustic focal points $P_{1a}$, $P_{2a}$ (or very closed to these points).

Such thermal increase can easily be detected by a thermal sensor: a thermal camera sensor, or a temperature sensible material. For example, a sheet of absorbent material that changes its colour locally in response to a local change of temperature can be used. Such material can be used at the surface S or in the plane of the beams, and eventually in combination with an optical camera.

A change of temperature can be determined in comparison to previous temperature values, or to a mean value. A relevant change of temperature due to ultrasound intensity concentrated inside the region V can be greater than a threshold, e.g. a threshold of 1°C or 0.5°C.

The local thermal increase at the acoustical focal points is then used to determine experimentally the exact position of the acoustic focal points $P_{1a}$, $P_{2a}$, and to implement the method of the invention.

The invention is to provide specific and optimised parameters so as to have the cavitation or acoustic effect and to superpose all the acoustic focal points of the transducers.

Unfortunately, for nonlinear transducers and/or nonlinear acoustic regimen, changing the amplitude of the signals influences or modifies the position of the acoustic focal points. Therefore, such tuning may be complex in practice.

To this end, the first and second signals are tuned to have an amplitude that is minimum to generate the cavitation effect (the acoustic effect), and the transducers must be also and simultaneously positioned to superpose the first and second acoustic focal points $P_{1a}$, $P_{2a}$ for that minimum amplitude of the signals. The cavitation effect is then localised inside the region V of the medium, around the intersection point I of the beams directions $D_1$, $D_2$. The cavitation effect is not localised outside of said region V.

The idea of the invention is that, for these optimised parameters:

- a small change of the positions of the transducers will cancel the acoustic effect; and
- a small reduction of the signals amplitude will cancel the acoustic effect. In that case, the acoustic effect occurs only inside a region (V) that has a minimum size.

The region (V) wherein the cavitation effect occurs has a reduced size. Its size may be smaller than 5 mm in all directions. Preferably, its size may be smaller than 2 mm in all directions.

The cavitation effect is itself non-linear: it appears suddenly if the parameters are adequately tuned, and disappears suddenly if the parameters are not adequately tuned. Thanks to this behaviour, an iterative method can be built to find the optimised best parameters values (signals and positions).

The device 1 may comprise a sensor 16 to detect the cavitation effect inside the medium. The sensor 16 may be an hydrophone sensor, a piezoelectric sensor, a fibre optic hydrophone sensor, a camera sensor, or any type of known sensor adapted to detect the cavitation.

The amplitude of the first and second signals can be defined by many different mathematical formulas. If $a_1$ is

the amplitude of the first signal $s_1$, and if $a_2$ is the amplitude of the second signal $s_2$, the amplitude a f both signals can be defined as:

$$a = \sqrt{(a_1^2 + a_2^2)/2}$$

However, every known method for norming, equalizing the signals can be applied to define an amplitude of the first and second signals. These definitions can also be applied to an unlimited number of signals.

The optimised parameters can be predetermined, for example with experiments on the device 1, and directly stored inside a memory of the control unit 30.

The optimised parameters can be determined via predetermined focal distances of a transducer for a plurality of signal amplitude provided to said transducer. The focal distances $d_1$, $d_2$ of both first and second transducers 11, 12 may be stored into the memory.

The optimised parameters can be determined from a mathematical model of the focal distance of a transducer versus amplitude of a signal provided to said transducer.

The control unit 30 may take into account a plurality of various factors that may influence the values of the predetermined optimised parameters or predetermined focal distances. For example, the factor may be the nature of the medium, the temperature of the medium, the temperature of the transducer.

The invention can also be used to determine optimised parameters so as to have a thermal effect and to superpose all the acoustic focal points of the transducers for this thermal effect. The thermal effect can be measured by the means disclosed above. The thermal effect is a local increase of temperature, for example during a specific duration.

The device and method may be used for thermoablation or thermal treatment inside an in vitro or in vivo medium. Such hyperthermia is well used for cancer treatment.

The device and method of the invention can therefore improve the hyperthermia, as the treated region V has a more reduced size.

**Figure 3** shows an embodiment of the device 1 comprising first and second transducers 11, 12, first and second displacement members 13, 14, and support member 10.

In this embodiment, the angle between the first and second beams directions is around 110°. An imaging transducer 12b situated between the first and second transducers to get images of the medium around the intersection point I.

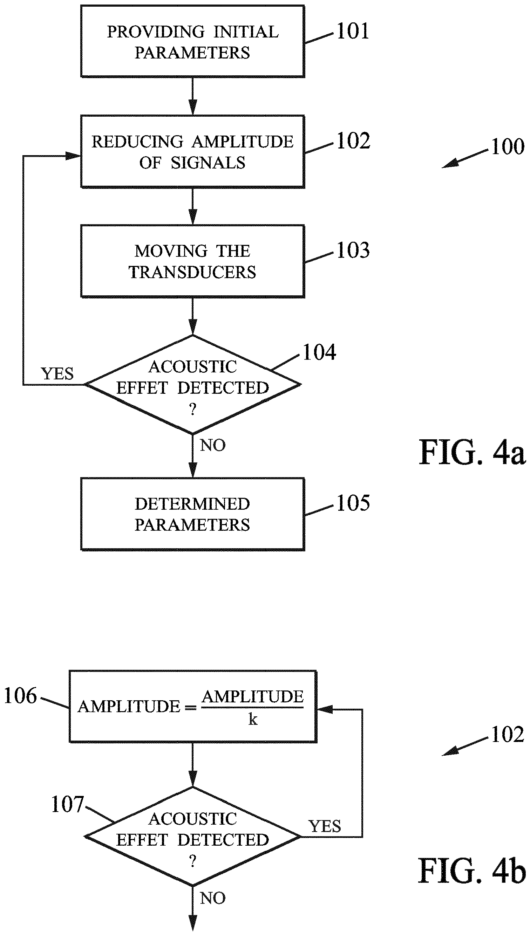

According to an embodiment of the device, the control unit 30 may determine the optimised parameters from an optimisation process 100 illustrated on **figure 4a** and having the following optimisation steps:

a) providing at step 101 initial parameters to move the first and second transducers by means of the first and second displacement members to a position and to generate first and second beams $B_1$, $B_2$ by means of said first and second transducers so that an acoustic effect is detected inside the medium,
b) reducing at step 102 an amplitude of the signals down to an amplitude level where the acoustic effect is not detected,
c) moving at step 103 the first and second transducers by means of the first and second displacement members to a plurality of test positions, each of said test positions corresponding to said position of the transducers wherein at least one of the first and second displacements is modified by a displacement increment (D),
d) if the acoustic effect is detected at step 104 at one of the test positions at step c), selecting said test position to be the position of the transducers, and repeating steps b) and c) (steps 102 and 103),
e) the determined parameters at step 105 are the signals and displacements tuned before the last reduction at step b).

A test position may correspond to the previous position of the transducers ($x_1$, $x_2$) wherein only one or any combination of the first or second displacements is increased or reduced by the displacement increment.

For example, if the first displacement of the test position is denoted by $x_1'$, and if the second displacement of the test position is denoted by $x_2'$, we can have any combination of:

$$x_1' = x_1 + D \quad ; \quad x_1' = x_1 - D \quad ;$$

$$x_2' = x_2 + D \ ; \quad x_2' = x_2 - D$$

These test positions enable to test if there is an acoustic effect at any position around the last or previous transducers position. This is a search procedure of optimal position, executed for a new amplitude at each loop of steps b) and c).

Therefore, the method is searching the optimal parameters that produce the acoustical effect for the minimal amplitude of the signals. This search method is executed with real signals and displacements of the transducers and is therefore very accurate.

During the optimisation steps, the first and second signals $s_1$, $s_2$ may have equal amplitudes. The method is therefore easier to implement.

The acoustic effect in the above optimisation steps and general method may be the cavitation effect, or the acoustic fountain effect, or the thermal increase effect.

For example, having a single acoustic fountain effect at the fluid surface for two emitted beams determines not only the position of the intersection point I, but also the position of the real superposed acoustic focal points $P_{1a}$, $P_{2a}$ for the used signals. A camera may be used as a sensor 16 to localise detects the acoustic fountain effect, and the control unit 30 may process the images from the camera to detect the acoustic effect. Consequently, the general method can be applied using the acoustic fountain effect.

The above method may be refined in doing between step d) and e) a step wherein: if the displacement increment D is not lower or equal than a minimum displacement increment $D_{min}$, the displacement increment D is reduced and the method is repeated steps b) and c).

Thanks to this feature, a more accurate optimal position of the first and second transducers 11, 13 (more accurate values of the first and second displacements $x_1$, $x_2$) can be determined.

Said minimum displacement increment $D_{min}$ may be equal to 0.1 mm, and preferably equal to 0.05 mm.

The displacement increment D may be equal to 0.5 mm, and preferably equal to 0.1 mm.

The determined position is therefore accurate.

The above method may be refined by implementing an iterative process at step b) (step 102) represented on **figure 4b**. The amplitude of the signals provided to the transducers is reduced step by step by the iterative process, wherein at each loop the amplitude is uptated by the following formula:

$$\texttt{amplitude = amplitude/k \quad and \quad 1<k<2}$$

where k is a reduction factor for decreasing the amplitude of the signals.

The reduction factor may be lower than 1.5, and preferably lower than 1.1.

The determined amplitude of the signals (i.e. the signals) is therefore accurate.

The displacement members 13, 14 may be activated to sequentially move the superposed acoustic focal points $P_{1a}$, $P_{2a}$ to a plurality of predetermined points inside a region of interest inside the medium. The region of interest may be determined by an image produced by the imaging transducer 12b.

It is well understood that the acoustic focal points $P_{1a}$, $P_{2a}$ may be moved by moving the first and second ultrasound means (transducers) relative to the medium or by moving the medium relative to the ultrasound means.

The control unit 30 comprises additional mechanical devices to move the medium or a portion of the medium relative to the transducers. For example, the mechanical devices comprises displacement drives according to one, two or a plurality of linear directions or one, two or another plurality of rotational directions, or any combination of linear or rotational directions. For example, the mechanical devices comprise three displacement drives according to three orthogonal directions X, Y, Z.

Alternatively the ultrasound means are able to produce a moveable beam of ultrasound wave, having a beam focused to a focal point having a controlled position inside the medium, without moving any mechanical parts. Such technique, is well known and use beam forming technology with an ultrasound mean having a plurality of transducers, each of them precisely controlled according a beam forming algorithm.

In other embodiments, the device 1 may comprise more than two ultrasound means (transducers) to generate a plurality of beams intersecting to an acoustic target point P. The region V has therefore a more reduced size and the cavitation inside this region V is increased and more stable.

In relation to **Figure 1,** the implementation of the above described device 1 in a **method** for delivering a substance inside a medium is now disclosed.

The method for delivering a substance inside a medium, wherein the substance is held by a delivery compound in a holding state, comprises the steps of:

- generating by first ultrasound means, a first beam of a first ultrasound wave inside said medium towards a first direction, said first beam being focused to a focal point inside said medium,

- generating by second ultrasound means, a second beam of a second ultrasound wave inside said medium towards a second direction, said second beam being substantially focused to said focal point, and wherein the second direction is inclined relative to the first direction for producing the cavitation inside a region of the medium around said focal point and for switching the delivery compound from the holding state to a releasing state inside said region.

Moreover, the method may implement the scanning of a region of interest inside the medium, by the added following steps:

- imaging the medium to determine a region of interest inside the medium,
- moving sequentially the focal point to a plurality of determined points inside the region of interest.

Firstly, the device and method may be used for drug delivery inside a body comprised inside the medium. The body is for example an animal or a human body. Thanks to the above-specified method the drug may be delivered inside the body to a predetermined region inside the body, and not somewhere else.
Moreover, the needed quantity of drug for the treatment of the predetermined region is greatly reduced compared to known methods without such delivery.
Such application has a great interest for the treatment of cancer tumor. The drug is released by the delivery compound only inside the tumor. The cancer tumor may be treated without releasing the drug everywhere inside the body. The drug is often harmful and toxic for organs inside the body. Many bad effects of a global treatment of the body may be therefore avoided.
Additionally, the quantity of drug injected inside the body is much smaller than the quantity for known methods.
Secondly, the device and method may be used for sonoporation or transfection applications, wherein plasmids are transferred inside a cell.
In the method, the substance comprises at least a plasmid. The substance may be a plasmid, a gene, or a plasmid graft on a liposome. The focal point is positioned near at least one cell. The cavitation phenomenon releases the substance and simultaneously opens the cell to transfer the plasmid inside said cell.
Tests were conducted to verify the efficiency of the combination of the transfection method and the present method for delivering a substance. Surprisingly, the cavitation generated for delivering the substance is also able to open the cells to transfer plasmids into the cells and without destroying said cells.
The test was done on mice. Two injections were done on these mice with RL cells so that 24 days after injection they have two tumors, each having a volume of at least 1000 mm$^3$.
The first tumor is insonified with dual confocal ultrasound beams as described above.
A substance siRNA Bcl2L1 comprising genes coupled to an Alexa Fluor is injected into the second tumor. The Alexa Fluor is an example of marker (fluorescence marker) adapted to detect the presence of siRNA added genes. The injected volume is 30 $\mu$l, and the substance concentration is of 7.5 $\mu$g/ml.
Then, the second tumor is also insonified with the same dual confocal ultrasound beams.
Both tumor are analysed to detect intracellular fluorescence. The second tumor comprises 16 % of fluorescent cells, that is to say 16 % of transfected cells. Higher percentages may be obtained.
Thirdly, the device and method may be used for destroying fat cells or adipocytes, inside a body. In that case, the substance may by a catalyst substance so that the adipocytes destruction is improved by the combination of the cavitation and the catalyst effects.

**Claims**

1. **A method** for determining parameters to generate ultrasound intensity localised inside a region (V) of a medium, wherein the method uses:

- a support member (10),
- first and second transducers (11, 12) linked to said support member and adapted to generate first and second focused beams ($B_1$, $B_2$) of ultrasound waves inside said medium in response to respective first and second signals ($s_1$, $s_2$), said beams being directed towards respective beams directions ($D_1$, $D_2$) that are substantially coplanar and inclined relative to each other of an angle comprised between 60° and 120°, and that intersects each other at an intersection point (I) inside the medium, said first and second transducers being different transducers, separated from each other, and
- first and second displacement members (13, 14) adapted to move at least one of the first and second transducers relative to the other transducer to a position determined by the first and second displacements ($x_1$, $x_2$) of the

respective first and second displacement members, said displacements being along different displacement directions,

**wherein** the parameters comprises the first and second signals ($s_1$, $s_2$) and the first and second displacements ($x_1$, $x_2$), and

**wherein** said parameters are optimised so that the first and second signals ($s_1$, $s_2$) have an amplitude that is minimum to generate an acoustic effect, said acoustic effect being localised only inside the region (V) of the medium around the intersection point (I) of the beams directions ($D_1$, $D_2$) and not localised outside of the region (V).

2. The method according to claim 1, further comprising the following successive optimisation steps:

**a)** providing initial parameters to move the first and second transducers by means of the first and second displacement members to a position and to generate first and second beams ($B_1$, $B_2$) by means of said first and second transducers so that an acoustic effect is detected inside the medium,

**b)** reducing an amplitude of the signals down to an amplitude level where the acoustic effect is not detected,

**c)** moving the first and second transducers by means of the first and second displacement members to a plurality of test positions, each of said test positions corresponding to said position of the transducers wherein at least one of the first and second displacements is modified by a displacement increment (D),

**d)** if the acoustic effect is detected at one of the test positions at step c), selecting said test position to be the position of the transducers, and repeating steps b) and c),

**e)** the determined parameters are the signals and displacements tuned before the last reduction at step b).

3. The method according to claim 2, comprising between step d) and e) a step wherein if the displacement increment (D) is not lower or equal than a minimum displacement increment ($D_{min}$), the displacement increment (D) is reduced and the method is repeated steps b) and c).

4. The method according to claim 3, wherein the minimum displacement increment ($D_{min}$) is equal to 0.1 mm, and preferably equal to 0.05 mm.

5. The method according to claim 2, wherein the displacement increment (D) is equal to 0.5 mm, and preferably equal to 0.1 mm.

6. The method according to claim 2, wherein the step b) is implemented by an iterative process wherein the amplitude of the signals is reduced at each loop by a division of the previous amplitude with a reduction factor (k) having a value comprised between one and two, said loop being repeated until the acoustic effect is not detected.

7. The method according to claim 6, wherein the reduction factor (k) is lower than 1.5, and preferably lower than 1.1.

8. The method according to any one of claim 1 to claim 7, wherein the acoustic effect is an effect chosen in a list comprising a cavitation effect localised inside the region (V) of the medium, and a single acoustic fountain effect for at least two beams and localised at an interface between a first and a second fluid of the medium, and a thermal increase effect.

9. **A device** for generating ultrasound intensity localised inside a region (V) of a medium, wherein the device comprises:

- a support member (10),
- first and second transducers (11, 12) linked to said support member and adapted to generate first and second focused beams ($B_1$, $B_2$) of ultrasound waves inside said medium in response to respective first and second signals ($s_1$, $s_2$), said beams being directed towards respective beams directions ($D_1$, $D_2$) that are substantially coplanar and inclined relative to each other of an angle comprised between 60° and 120°, and that intersects each other at an intersection point (I) inside the medium, said first and second transducers being different transducers, separated from each other, and
- first and second displacement members (13, 14) adapted to move at least one of the first and second transducers relative to the other transducer to a position determined by the first and second displacements ($x_1$, $x_2$) of the respective first and second displacement members, said displacements being along different displacement directions,

**wherein** the parameters comprises the first and second signals ($s_1$, $s_2$) and the first and second displacements ($x_1$, $x_2$), and

**wherein** the device further comprises a control unit (30) adapted to provide the parameters to move the first and second transducers by means of the first and second displacement members to a position and to generate first and second beams ($B_1$, $B_2$) by means of said first and second transducers, and

**wherein** the parameters are optimised so that the first and second signals ($s_1$, $s_2$) have an amplitude that is minimum to generate an acoustic effect, said acoustic effect being localised only inside the region (V) of the medium around the intersection point (I) of the beams directions ($D_1$, $D_2$) and not localised outside of the region (V).

10. The device according to claim 9, wherein the control unit (30) further comprises a memory and determines the parameters from at least a predetermined focal distance ($d_1$, $d_2$) of a transducer stored into the memory for a plurality of signal amplitude.

11. The device according to claim 9, wherein the control unit (30) determines the parameters from a predetermined mathematical model of a focal distance ($d_1$, $d_2$) of a transducer versus amplitude of a signal provided to the transducer.

12. The device according to claim 9, wherein the first and second transducers (11, 12) are high intensity focused ultrasound (HIFU) transducers.

13. The device according to claim 9, further comprising a sensor (16) adapted to detect the acoustic effect inside the medium.

14. The device according to claim 13, wherein the sensor (16) is chosen in a list comprising an hydrophone sensor, a piezoelectric sensor, a fibre optic hydrophone, a thermal sensor, and a camera sensor.

15. The device according to claim 9, wherein the angle is comprised between 70° and 110°.

16. The device according to claim 9, wherein each of the displacement directions are perpendicular to one of the beam directions ($D_1$, $D_2$).

17. The device according to claim 9, wherein the region (V) has a size determined by the parameters, said size being smaller than 5 mm in all directions, and preferably smaller than 2 mm in all directions.

18. The device according to any one of the claims 9 to 17, wherein the control unit (30) determines the parameters to generate an ultrasound pressure inside the region (V) of the medium executing the following optimisation steps:

a) providing initial parameters to move the first and second transducers by means of the first and second displacement members to a position and to generate first and second beams ($B_1$, $B_2$) by means of said first and second transducers so that an acoustic effect is detected inside the medium,
b) reducing an amplitude of the signals down to an amplitude level where the acoustic effect is not detected,
c) moving the first and second transducers by means of the first and second displacement members to a plurality of test positions, each of said test positions corresponding to said position of the transducers wherein at least one of the first and second displacements is modified by a displacement increment (D),
d) if the acoustic effect is detected at one of the test positions at step c), selecting said test position to be the position of the transducers, and repeating steps b) and c),
e) the determined parameters are the signals and displacements tuned before the last reduction at step b).

19. The device according to any one of the claim 9 to the claim 18, wherein the acoustic effect is an effect chosen in the list comprising a cavitation effect localised inside the region (V) of the medium, and a single acoustic fountain effect for at least two beams and localised at an interface between a first and a second fluid of the medium, and a thermal increase effect.

FIG. 1

FIG. 2

FIG. 3

FIG. 4a

FIG. 4b

Europäisches
Patentamt
European
Patent Office
Office européen
des brevets

# EUROPEAN SEARCH REPORT

Application Number

EP 12 30 5278

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X<br>Y | US 5 501 655 A (ROLT KENNETH D [US] ET AL) 26 March 1996 (1996-03-26)<br>* abstract *<br>* column 3, line 65 - column 7, line 30 *<br>----- | 9,12-19<br><br>10,11 | INV.<br>A61N7/02 |
| X | US 6 428 532 B1 (DOUKAS APOSTOLOS [US] ET AL) 6 August 2002 (2002-08-06)<br>* abstract *<br>* column 2, line 5 - line 49 *<br>* column 3, line 11 - line 42 *<br>* column 5, line 10 - column 6, line 58 *<br>* column 7, line 62 - column 9, line 50 *<br>----- | 9,12,16,<br>17,19 | |
| X | WO 2009/045411 A2 (INSPIRED SURGICAL TECHNOLOGIES [US]; OOSTING KENNETH [US]; SNOOK AMY [) 9 April 2009 (2009-04-09)<br>* abstract *<br>* paragraph [0010] *<br>* paragraph [0018] - paragraph [0026] *<br>* paragraph [0042] - paragraph [0049] *<br>* paragraph [0074] - paragraph [0080] *<br>----- | 9,13 | |
| Y | WO 00/78232 A1 (TRANSURGICAL INC [US]) 28 December 2000 (2000-12-28)<br>* abstract *<br>* page 7, line 6 - page 10, line 19 *<br>* page 13, line 3 - page 17, line 10 *<br>----- | 10,11 | TECHNICAL FIELDS SEARCHED (IPC)<br><br>A61N |
| E | WO 2012/028898 A1 (INST NAT SANTE RECH MED [FR]; CHAPELON JEAN-YVES [FR]; LAFON CYRIL [FR]) 8 March 2012 (2012-03-08)<br>* the whole document *<br>----- | 9 | |

The present search report has been drawn up for all claims

| Place of search | Date of completion of the search | Examiner |
|---|---|---|
| Munich | 3 August 2012 | Beck, Ewa |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
...............................................................
& : member of the same patent family, corresponding document

EPO FORM 1503 03.82 (P04C01)

**ANNEX TO THE EUROPEAN SEARCH REPORT
ON EUROPEAN PATENT APPLICATION NO.** EP 12 30 5278

This annex lists the patent family members relating to the patent documents cited in the above-mentioned European search report.
The members are as contained in the European Patent Office EDP file on
The European Patent Office is in no way liable for these particulars which are merely given for the purpose of information.

03-08-2012

| Patent document cited in search report | | Publication date | Patent family member(s) | | Publication date |
|---|---|---|---|---|---|
| US 5501655 | A | 26-03-1996 | US | 5501655 A | 26-03-1996 |
| | | | WO | 9319705 A1 | 14-10-1993 |
| US 6428532 | B1 | 06-08-2002 | NONE | | |
| WO 2009045411 | A2 | 09-04-2009 | AU | 2008307601 A1 | 09-04-2009 |
| | | | CA | 2701628 A1 | 09-04-2009 |
| | | | CN | 101883608 A | 10-11-2010 |
| | | | EP | 2219731 A2 | 25-08-2010 |
| | | | JP | 2010540163 A | 24-12-2010 |
| | | | KR | 20100099103 A | 10-09-2010 |
| | | | US | 2009088625 A1 | 02-04-2009 |
| | | | WO | 2009045411 A2 | 09-04-2009 |
| WO 0078232 | A1 | 28-12-2000 | AU | 5496700 A | 09-01-2001 |
| | | | WO | 0078232 A1 | 28-12-2000 |
| WO 2012028898 | A1 | 08-03-2012 | NONE | | |

EPO FORM P0459

For more details about this annex : see Official Journal of the European Patent Office, No. 12/82

**REFERENCES CITED IN THE DESCRIPTION**

**Patent documents cited in the description**

- WO 2008018019 A